# EUROPÄISCHE PATENTSCHRIFT

(11) **EP 1 320 449 B1**
(45) Veröffentlichungstag und Bekanntmachung des Hinweises auf die Patenterteilung: **27.09.2006**
(21) Anmeldenummer: 01976089.1
(22) Anmeldetag: 16.08.2001
(51) Int. Cl.: B29B 7/60, B01F 15/04, G05D 11/13, C07C 409/24

(54) **VERFAHREN ZUR KONTINUIERLICHEN HERSTELLUNG VON STOFF- UND REAKTIONSGEMISCHEN UND VORRICHTUNG ZU SEINER DURCHFÜHRUNG**
METHOD FOR THE CONTINUOUS PRODUCTION OF MIXTURES OF SUBSTANCES AND REACTION MIXTURES AND A DEVICE FOR CARRYING OUT SAID METHOD
PROCEDE DE PRODUCTION CONTINUE DE MELANGES DE SUBSTANCES OU DE MELANGES REACTIONNELS ET DISPOSITIF POUR LA MISE EN OEUVRE DE CE PROCEDE

(30) Priorität: 29.09.2000 DE 10048513
(43) Veröffentlichungstag der Anmeldung: 25.06.2003
(73) Patentinhaber: Degussa AG, 40474 Düsseldorf (DE)
(72) Erfinder: STEINBERGER, Michael, A-9220 Velden (AT); MIKLAUTSCH, Josef, A-9500 Villach (AT)
(86) Internationale Anmeldenummer: PCT/EP2001/009454
(87) Internationale Veröffentlichungsnummer: WO 2002/026459

(56) Entgegenhaltungen:
- EP-A- 0 026 874
- EP-A- 0 641 777
- DE-A- 3 638 552
- DE-A- 3 800 788
- US-A- 3 817 658
- US-A- 4 019 653
- US-A- 4 176 672
- US-A- 4 440 314

## Beschreibung

Die Erfindung richtet sich auf ein Verfahren zur kontinuierlichen Herstellung einer wässrigen Gleichgewichtsperoxycarbonsäurelösung, umfassend Zusammenbringen der das Stoffgemisch bildenden einzelnen Komponenten in mengenproportionalem Verhältnis und Stehenlassen des Stoffgemischs in einem Behälter bis zur Einstellung des gewünschten Umsatzes, insbesondere der Einstellung des Gleichgewichtszustandes von eine Gleichgewichtszusammensetzung bildenden Komponenten. Die Erfindung richtet sich ferner auf eine Vorrichtung zur Durchführung des Verfahrens, wobei die Vorrichtung als on-site-Anlage errichtet werden kann und sich in besonderer Weise zur Herstellung von Peroxycarbonsäurelösungen eignet.

Bisher werden chemische Reaktionen in der Regel in Reaktoren unterschiedlichster Konstruktionsweise durchgeführt. Die Reaktionskomponenten werden dabei entweder nacheinander oder simultan dem Reaktor zugeführt und dort gemischt und umgesetzt. Nach erfolgter Durchmischung und Abklingen der Reaktion wird das Produkt, sofern sich keine besondere Aufarbeitung anschließt, in Versandbehälter oder Vorratsbehälter abgefüllt. Wesentliche Nachteile dieses Verfahrens sind die hohen Investitionskosten, da einerseits ggf. in besonderer Weise konstruierte Reaktoren mit entsprechendem Füllvolumen erforderlich sind und zusätzlich in vielen Fällen, beispielsweise beim Einsatz und/oder der Bildung von Produkten mit hohem Oxidationspotential sowie leicht brennbaren und/oder explosionsfähigen Stoffen oder Stoffgemischen aus sicherheitstechnischen Überlegungen ein erheblicher Aufwand für sicherheitstechnische Einrichtungen und deren Steuerung getrieben werden muss. Weitere Nachteile der konventionellen Vorrichtungen sind die langen Chargenzeiten sowie der hohe Aufwand für die Reinigung der gesamten Anlage im Falle eines Produktwechsels.

Aus dem US-Patent 5,887,975 ist ein Mehrkomponenten-inline-Farbmischsystem bekannt, in welchem die einzelnen Komponenten der Farbe nicht in einem Mischbehälter gemischt und homogenisiert werden, sondern in welchem die einzelnen Komponenten pulsweise und hintereinander in mengenproportionalem Verhältnis durch eine reduzierende Rohrverzweigung geleitet werden und die hintereinander in Pulssegmenten in der Austrittsleitung strömenden einzelnen Komponenten unmittelbar vor dem Versprühen der Farbe in einem Integrator zusammengeführt und in einem Static-Mixer gemischt werden. Durch die Anordnung ist es möglich, auch miteinander reaktionsfähige Komponenten so zu dosieren und zu mischen, dass die eigentliche Reaktion erst nach dem Versprühen der Farbe eintritt.

Das zuvor gewürdigte Verfahren ist zur Herstellung von Stoff- und Reaktionsgemischen, welche letztlich in einem Behälter gelagert werden können, weniger geeignet, weil die pulsweise Dosierung zu langen Chargenzeiten führt. Zudem ist das Verfahren aus sicherheitstechnischen Aspekten heraus dann nicht anwendbar, wenn bestimmte Kombinationen der das Gemisch bildenden Komponenten selbst explosiv sind. Durch den Transport der Komponenten hintereinander durch die gleiche Leitung sind solche Probleme nicht vermeidbar.

Bei der Herstellung von Gleichgewichtsperoxycarbonsäuren beispielsweise ist von wesentlicher Bedeutung, die einzelnen Komponenten in der richtigen Reihenfolge zu dosieren, da sonst explosionsfähige Gemische entstehen können. Im Falle der Gleichgewichtsperoxycarbonsäuren ist dafür Sorge zu tragen, dass hochkonzentriertes wässriges Wasserstoffperoxid als letzte Komponente mit einem Gemisch aus einer Carbonsäure, Wasser und einem Mineralsäurekatalysator in Kontakt gebracht wird.

DE 36 38 552 beschreibt ein Verfahren zur Herstellung von Peroxosäuren durch Umsetzung der entsprechenden Carbonsäure mit Wasserstoffperoxid unter Katalyse mit Mineralsäuren, bei dem man wässrige Lösungen von Carbonsäure, Katalysator und Wasserstoffperoxid gleichzeitig über einen statischen Mischer in einem als Reaktionsgefäß ausgelegten Standcontainer zusammengibt und unter Bildung von Peroxosäure reagieren lässt.

Aufgabe der Erfindung ist es demgemäß, ein Verfahren zur sicheren Herstellung von wässrigen Peroxycarbonsäurelösungen aufzuzeigen, womit Stoffgemische, welche miteinander unter Freisetzung mäßiger Reaktionswärme reaktionsfähige Komponenten enthalten, kontinuierlich hergestellt werden können, wobei die eigentliche Reaktion erst im Versandbehälter oder einem für die weitere Verwendung bereit gestellten Zwischenbehälter stattfindet. Gemäß einer weiteren Aufgabe sollte das Verfahren zuverlässig geregelt werden können. Gemäß einer weiteren Aufgabe der Erfindung sollte eine Vorrichtung zur Durchführung des Verfahrens bereit gestellt werden. Die Vorrichtung sollte einfach aufgebaut sein und sich als on-site-Anlage direkt bei den Verbrauchern des Stoff- bzw. Reaktionsgemischs errichten lassen, um so den Transport von Gleichgewichtsperoxycarbonsäuren höherer Konzentration zu vermeiden.

Diese und weitere Aufgaben, wie sie sich aus der weiteren Beschreibung ergeben, lassen sich durch das erfindungsgemäße Verfahren lösen. Gefunden wurde ein Verfahren zur kontinuierlichen Herstellung einer wässrigen Gleichgewichtsperoxycarbonsäurelösung, umfassend Zusammenbringen der die Gleichgewichtsperoxycarbonsäurelösung bildenden einzelnen Komponenten aus der Reihe niederer Carbonsäure, insbesondere Essigsäure, wässrigem Wasserstoffperoxid, Wasser und Mineralsäurekatalysator und Stehenlassen des Stoffgemischs in einem Behälter bis zur Einstellung eines gewünschten Umsatzes, das dadurch gekennzeichnet ist, dass man die einzelnen Komponenten Vorratsbehältern oder Verteilernetzen entnimmt und kontinuierliche Ströme der einzelnen Komponenten bildet, jeden Komponentenstrom über eine eine Massen- oder Volumen-Durchflussmessvorrichtung und ein Regelorgan zur Regelung des Durchflusses umfassende Regelstrecke leitet, die Mengenströme der einzelnen Komponenten unter Bezugnahme auf den Mengenstrom einer ersten Komponente mengenproportional regelt und die geregelten Mengenströme der Komponenten des Stoffgemischs unmittelbar oder nach vollständigem oder teilweisem Zusammenführen einzelner Mengenströme in einen Aufnahmebehälter einbringt und den Durchfluss des aus den einzelnen Strömen gebildeten Gesamtstroms oder die Gesamtmenge der in den Behälter eingebrachten Einzel- oder Teilströme misst und mit der Summe der einzelnen Ströme abgleicht. Die Unteransprüche richten sich auf bevorzugte Ausführungsformen dieses Verfahrens.

Das erfindungsgemäße Verfahren richtet sich auf die Herstellung von wässrigen Gleichgewichtsperoxycarbonsäurelösungen, wie wässrige Gleichgewichtsperoxyessigsäurelösungen. Hierbei wird aus einer oder mehreren organischen Carbonsäuren, Wasser, einem Mineralsäurekatalysator und wässrigem Wasserstoffperoxid ein Stoffgemisch gebildet, aus welchem sich beim Stehenlassen die Gleichgewichtsperoxycarbonsäurelösung bildet. Die chemische Reaktion erfolgt demgemäß nicht in einem speziellen Reaktor oder großvolumigem Behälter, sondern in dem das Stoffgemisch aufnehmenden Behälter. Der besondere Vorteil der erfindungsgemäßen Verfahrensweise besteht darin, dass ein in sicherheitstechnischer Hinsicht nicht unproblematischer Stoff, wie eine höherkonzentrierte wässrige Peroxycarbonsäurelösung, on-site am Ort des Bedarfs hergestellt werden kann. Damit erübrigt sich ein ggf. aufwendiger Transport; zusätzlich ist es möglich, das Reaktionsgemisch in einer solchen Konzentration herzustellen, die aus sicherheitstechnischer Sicht einem Transport entgegenstehen würde.

Zur Herstellung der erfindungsgemäß erhältlichen Peroxycarbonsäurelösungen werden vorzugsweise wasserlösliche Carbonsäuren oder Dicarbonsäuren mit 1 bis 6 C-Atomen eingesetzt. Es ist möglich, reine Carbonsäuren oder Carbonsäuregemische zu verwenden, und derartige Gemische können zusätzlich auch eine wasserunlösliche Carbonsäure, d. h. eine Carbonsäure mit mehr als 6 C-Atomen enthalten. Besonders bevorzugt werden Peroxyessigsäurelösungen, insbesondere GleichgewichtsPeroxyessigsäurelösungen nach dem erfindungsgemäßen Verfahren hergestellt. Um die oxidative oder Desinfektionswirkung von Peroxyessigsäure zu erhöhen, ist es zweckmäßig, dem Stoffgemisch zusätzlich Ameisensäure oder eine Quelle für Ameisensäure zuzusetzen. Als Katalysator für die Gleichgewichtseinstellung eignen sich Ameisensäure oder Mineralsäuren, wie insbesondere Schwefelsäure, Phosphorsäure und besonders bevorzugt Polyphosphorsäure. Wasserstoffperoxid wird in unterschiedlicher Konzentration eingesetzt, vorzugsweise in einer Konzentration von 30 bis 85 Gew.-%, inbesondere 50 bis 85 Gew.-%. Der sicherheitstechnische Aspekt ist bei der Herstellung von Peroxycarbonsäuren von ausserordentlicher Bedeutung, da organische Carbonsäuren mit Wasserstoffperoxid hoher Konzentration gemischt werden.

Durch das erfindungsgemäße Verfahren ist es möglich, sowohl die Mengenverhältnisse der einzelnen Komponenten des Stoffgemischs und des daraus gebildeten Reaktionsgemischs als auch die Dosierreihenfolge exakt einzuhalten und damit die Bildung explosibler Gemische sicher zu vermeiden.

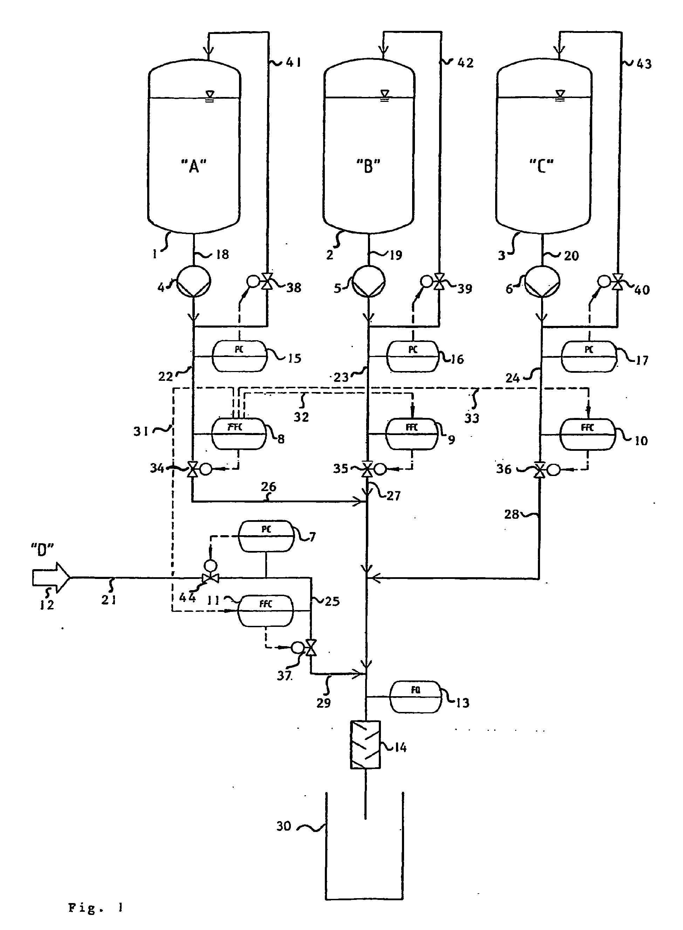

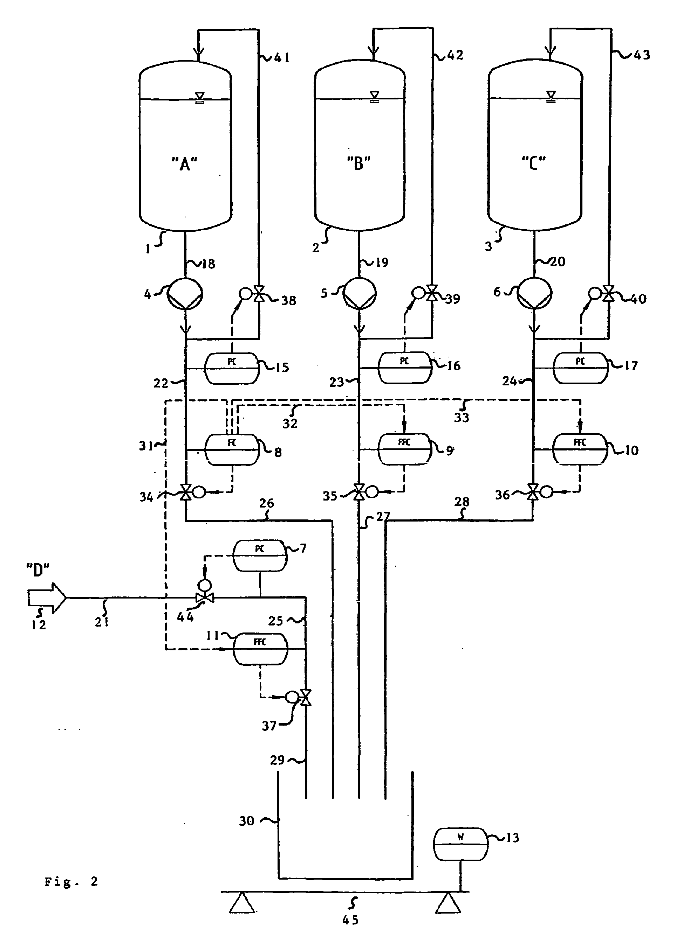

Das erfindungsgemäße Verfahren wird anhand der Verfahrenschemata gemäß Figur 1 und 2 weiter erläutert.

Die einzelnen Komponenten des herzustellenden Stoffgemischs, wie A, B und C werden den Vorratsbehältern 1, 2 und 3 entnommen; alternativ können ein oder mehrere Komponenten auch aus am Ort der Herstellung verfügbaren Verteilernetzen entnommen werden, beispielsweise die Komponente D aus dem Verteilernetz 12. Bei den Einzelkomponenten kann es sich hierbei sowohl um reine Stoffe als auch um Stoffgemische handeln. Auch wenn in den Figuren 1 und 2 die Stoffe A, B, C und D zum Einsatz gelangen, ist es möglich, auch Stoffgemische aus weniger oder mehr Komponenten zu erzeugen; in diesem Fall wird die erforderliche Vorrichtung zur Herstellung an die Anzahl der Komponenten angepasst. Die aus den Vorratsbehältern oder/und Verteilernetzen über Leitungen (18, 19, 20 und 21) entnommenen Komponenten werden mittels Pumpen (4, 5 und 6) oder durch vorhandenen Druck im Falle von Verteilernetzen oder mittels anderer Mittel zur Erzeugung eines wirksamen Drucks in eine für jede Komponente vorhandene Regelstrecke (22, 23, 24 und 25) geleitet. Zur Schaffung konstanter Dosierbedingungen kann der Vordruck vor der eigentlichen Regelstrecke mittels bekannter Mittel zur Druckregelung (15, 16, 17 und 7) kontrolliert werden. Beispielsweise kann die Druckregelung im Falle der Vorratsbehälter mittels einer Umlaufleitung (41, 42 und 43) mit darin befindlichem Regelorgan (38, 39 und 40), wobei eine Steuerleitung von der Vorrichtung für die Druckregelung (15, 16 und 17) zu den Regelorganen (38, 39 und 40) führt, eingestellt werden. Der Vordruck der Komponente aus dem Verteilernetz kann mittels des Druckreglers (7), dessen Steuerleitung ein Regelorgan (44) ansteuert, geregelt und konstant gehalten werden. Die Regelstrecke für jede Komponente umfasst eine Leitung (22, 23, 24 und 25), eine Durchflussmessvorrichtung (8, 9, 10 und 11) und ein Regelorgan, insbesondere ein Regelventil (34, 35, 36 und 37), wobei eine Steuerleitung aus der jeweiligen Durchflussmessvorrichtung das Regelorgan ansteuert. Zur Durchflussmessung eignen sich bekannte Vorrichtungen zur Erfassung der Masse oder des Volumens. Im Falle des Volumenstroms ist, wenn Qualitätsschwankungen vermieden werden sollen, auf Temperaturkonstanz zu achten oder der Einfluss der Temperatur auf das Volumen zu berücksichtigen. Alternativ zu der Kombination aus Durchflussmessung und Regelventil können Drehzahl geregelte Pumpen oder Dosierpumpen zum Einsatz gelangen, welche gemeinsam die Funktionen der vorgenannten Geräte erfüllen. Die Mengenströme der Komponenten A, B und C werden unter Bezugnahme auf einen Mengenstrom, im allgemeinen dem Mengenstrom der Komponente mit dem größten Mengendurchfluss (in den Figuren handelt es sich um den Mengenstrom der Komponente A), mengenproportional geregelt. Demgemäß greifen Steuerleitungen (31, 32 und 33) von der Vorrichtung für die Merigendurchflussmessung der Komponente A auf die Vorrichtung für die Durchflussmessung (9, 10 und 11) der Komponenten B, C und D. Die Steuereinheit zur Abstimmung der Messungen und der Regler untereinander, wobei alle relevanten Daten in Form von Rezepten im System hinterlegt sind, wird in den Figuren nicht gezeigt. Vorzugsweise wird die Steuerung der Anlage mittels Prozessleittechnik realisiert. Der Begriff "mengenproportionale Regelung" umfasst auch eine mechanische Einstellung eines Volumenverhältnisses der einzelnen Komponenten.

An die Regelstrecke jeder Komponente schließt sich eine Abgangsleitung (26, 27, 28 und 29) an. Gemäß Figur 1 werden die Komponentenströme hintereinander zusammengeführt, gemäß Figur 2 werden die Mengenströme einzeln in einen Aufnahmebehälter (30) geleitet. In der Ausführungsform gemäß Figur 1 werden die einzelnen mengenproportional geregelten Komponentenströme hintereinander zusammengeführt, und der Gesamtstrom gelangt in den Aufnahmebehälter (30). Um die Homogenität in dem Aufnahmebehälter zu gewährleisten, können die vereinigten Teilströme über ein Mischorgan (14), welches vorzugsweise in Form eines Static-Mixers ausgeführt ist, geführt. Unter dem Aspekt der Sicherheitstechnik und der Qualitätssicherung, ist es zweckmäßig, die Dosiergenauigkeit dadurch zu kontrollieren, dass auch der Durchfluss des Gesamtstromes nach der Vereinigung der einzelnen Komponentenströme mittels einer Durchflussmessvorrichtung (13) gemessen wird. Aus sicherheitstechnischer Sicht ist in vielen Fällen von Bedeutung, in welcher Reihenfolge die einzelnen Komponentenströme zusammengeführt werden. Diese Reihenfolge ist für die zuvor erörterte Herstellung von Peroxycarbonsäurelösungen von besonderer Bedeutung.

Sollte durch Turbulenzen, Diffusion oder ein im Aufnahmebehälter angeordnetes Rührwerk eine ausreichende Durchmischung gewährleistet sein, kann auf das. Vereinigen der Teilströme bzw. auch auf das Vorschalten eines Mischorgans verzichtet werden. In diesem Fall kann eine Kontroll-Mengenmessung mittels beispielsweise einer Waage (45) erfolgen. Gemäß einer bevorzugten Ausführungsform wird der Durchfluss des gebildeten Gesamtstroms (Figur 1) oder die Gesamtmenge der in den Behälter eingebrachten Einzel-oder Teilströme oder des Gesamtstroms (Figur 2) gemessen und mit der Summe der Einzelströme abgeglichen.

Ein weiterer Gegenstand der Erfindung ist eine Vorrichtung zur kontinuierlichen Herstellung von Stoff- oder Reaktionsgemischen zur Durchführung des zuvor beschriebenen Verfahrens. Diese Vorrichtung umfasst Vorratsbehälter zur kontinuierlichen Herstellung von Stoff- oder Reaktionsgemischen gemäß einem der Ansprüche 1 bis 5, umfassend Vorratsbehälter oder Verteilernetze für die einzelnen Komponenten des Stoffgemischs, Vorrichtungen zum Dosieren der einzelnen Komponenten und einen Aufnahmebehälter und ist dadurch gekennzeichnet, dass eine aus jedem Vorratsbehälter (1, 2, 3) oder Verteilernetz (12) abgehende Leitung als Regelstrecke (22 bis 25) ausgebildet ist, jede Regelstrecke eine Durchflussmessvorrichtung (8 bis 11) und ein Regelorgan (34 bis 37) zur Regelung des Durchflusses aufweist und eine Reglereinheit mit Steuerleitungen (31, 32, 33) eine mengenproportionale Dosierung der Komponenten ermöglicht, und die den Regelstrecken nachgeschalteten Leitungen (26 bis 29) unmittelbar oder nach Zusammenführen einzelner oder aller Leitungen in einen Aufnahmebehälter (30) münden. Diese Vorrichtung sowie bevorzugte Ausführungen hierfür wurden bereits bei der Beschreibung des erfindungsgemäßen Verfahrens erörtert,

Das erfindungsgemäße Verfahren und die hierfür konzipierte Vorrichtung gestatten in einfacher und zuverlässiger Weise die Herstellung auch komplexer Stoff- und Reaktionsgemische ohne die Verwendung ggf. aufwendiger Reaktionsapparate. Durch die Reihenfolge, in welcher die mengengeregelten Teilströme der einzelnen Komponenten zusammengeführt werden, lässt sich sicherstellen, dass die Entstehung gefährlicher Komponentengemische vermieden wird.

## Patentansprüche

1. Verfahren zur kontinuierlichen Herstellung einer wässrigen Gleichgewichtsperoxycarbonsäurelösung, umfassend Zusammenbringen der die Gleichgewichtsperoxycarbonsäurelösung bildenden einzelnen Komponenten aus der Reihe niederer Carbonsäure, insbesondere Essigsäure, wässrigem Wasserstoffperoxid, Wasser und Mineralsäurekatalysator und Stehenlassen des Stoffgemischs in einem Behälter bis zur Einstellung eines gewünschten Umsatzes,
**dadurch gekennzeichnet,**
**dass** man die einzelnen Komponenten Vorratsbehältern oder Verteilernetzen entnimmt und kontinuierliche Ströme der einzelnen Komponenten bildet, jeden Komponentenstrom über eine eine Massen- oder Volumen-Durchflussmessvorrichtung und ein Regelorgan zur Regelung des Durchflusses umfassende Regelstrecke leitet, die Mengenströme der einzelnen Komponenten unter Bezugnahme auf den Mengenstrom einer ersten Komponente mengenproportional regelt und die geregelten Mengenströme der Komponenten des Stoffgemischs unmittelbar oder nach vollständigem oder teilweisem Zusammenführen einzelner Mengenströme in einen Aufnahmebehälter einbringt und den Durchfluss des aus den einzelnen Strömen gebildeten Gesamtstroms oder die Gesamtmenge der in den Behälter eingebrachten Einzel-oder Teilströme misst und mit der Summe der einzelnen Ströme abgleicht.

2. Verfahren nach Anspruch 1,
**dadurch gekennzeichnet,**
**dass** man zur Durchflussmessung eine Vorrichtung zur Massendurchflussmessung oder eine Dosierpumpe verwendet.

3. Verfahren nach Anspruch 1 oder 2,
**dadurch gekennzeichnet,**
**dass** man die aus den Vorratsbehältern abgezogenen Ströme mittels Pumpen oder durch vorhandenen Vordruck über die Regelstrecke leitet.

4. Verfahren nach einem der Ansprüche 1 bis 3,
**dadurch gekennzeichnet,**
**dass** man zur Konstanthaltung der Regelbedingungen den Vordruck des jeweiligen Komponentenstromes vor der jeweiligen Regelstrecke auf einen konstanten Wert einstellt.

5. Verfahren nach einem der Ansprüche 1 bis 4,
**dadurch gekennzeichnet,**
**dass** man die mengenproportionalen Komponentenströme hintereinander zusammenführt und gebildete Teilströme oder den gebildeten Gesamtstrom über Mischorgane, insbesondere Static-Mixer, homogenisiert.

6. Vorrichtung zur kontinuierlichen Herstellung von Gleichgewichtsperoxycarbonsäurelösungen gemäß einem der Ansprüche 1 bis 5, umfassend Vorratsbehälter oder Verteilernetze für die einzelnen Komponenten der Gleichgewichtslösung, Vorrichtungen zum Dosieren der einzelnen Komponenten und einen Aufnahmebehälter,
**dadurch gekennzeichnet,**
**dass** eine aus jedem Vorratsbehälter (1, 2, 3) oder Verteilernetz (12) abgehende Leitung als Regelstrecke (22 bis 25) ausgebildet ist, jede Regelstrecke eine Durchflussmessvorrichtung (8 bis 11) und ein Regelorgan (34 bis 37) zur Regelung des Durchflusses aufweist und eine Reglereinheit mit Steuerleitungen (31, 32, 33) eine mengenproportionale Dosierung der Komponenten ermöglicht, und die den Regelstrecken nachgeschalteten Leitungen (26 bis 29) unmittelbar oder nach Zusammenführen einzelner oder aller Leitungen in einen Aufnahmebehälter (30) münden und zwischen den Vorratsbehältern und der dazugehörigen Regelstrecke jeweils eine Pumpe (4 bis 6) und Vorrichtungen zur Konstanthaltung des Vordrucks (38 bis 43) vor der Regelstrecke angeordnet sind.

7. Vorrichtung nach Anspruch 6,
**dadurch gekennzeichnet,**
**dass** die Regelstrecke zur Durchflussmessung einen Massendurchflussmesser oder eine Dosierpumpe enthält..

8. Vorrichtung nach einem der Ansprüche 6 oder 7,
**dadurch gekennzeichnet,**
**dass** sie zwecks Abstimmung der Messungen und Regler untereinander Vorrichtungen zur Prozessleittechnik aufweist.

9. Vorrichtung nach einem der Ansprüche 6 bis 8,
**dadurch gekennzeichnet,**
**dass** sie in einer Leitung, resultierend aus der Zusammenführung der Leitungen (26) bis (29), ein Mischorgan (14), insbesondere Static-Mixer, enthält.

## Claims

1. A process for the continuous production of an aqueous equilibrium solution of a peroxycarboxylic acid, comprising bringing together the individual components forming the peroxycarboxylic acid equilibrium solution, in particular acetic acid, aqueous hydrogen peroxide, water and mineral-acid catalyst, and allowing the mixture of substances to stand in a container until a desired conversion has been established,
**characterised in that**
the individual components are withdrawn from storage containers or from distribution networks and continuous streams of the individual components are formed, each component stream is conveyed via a controlled system comprising a mass-flow or volume-flow measuring device and a regulating element for regulating the rate of flow, the flow-rates of the individual components are regulated in quantitatively proportional manner with reference to the flow-rate of a first component and the regulated flow-rates of the components of the mixture of substances are introduced into a receiving container, immediately or after individual flow-rates have been completely or partially conducted together, and the rate of flow of the total stream that has formed or the total quantity of the individual streams or partial streams introduced into the container or of the total stream is measured and verified against the sum of the individual streams.

2. Process according to Claim 1,
**characterised in that**
a device for mass-flow measurement or a metering pump is used for the flow measurement.

3. Process according to Claim 1 or 2,
**characterised in that**
the streams drawn off from the storage containers are conveyed along the controlled system by means of pumps or by available primary pressure.

4. Process according to one of Claims 1 to 3,
**characterised in that**
the primary pressure of the respective component stream upstream of the respective controlled system is adjusted to a constant value to keep the regulating conditions constant.

5. Process according to one of Claims 1 to 4,
**characterised in that**
the component streams are combined successively in proportion to quantity and partial streams that are formed or the total stream that is formed are homogenized with the aid of mixing elements, in particular static mixers.

6. A device for the continuous production of an aqueous equilibrium solution of a peroxycarboxylic acid according to one of Claims 1 to 5, comprising storage containers or distribution networks for the individual components of the equilibrium solution, devices for metering the individual components and a receiving container,
**characterised in that**
a line branching off from each storage container (1, 2, 3) or distribution network (12) takes the form of a controlled system (22 to 25), each controlled system exhibits a flow-measuring device (8 to 11) and a regulating element (34 to 37) for regulating the rate of flow and a regulator unit with control lines (31, 32, 33) enables a quantity-based proportional metering of the components, and the lines (26 to 29) connected downstream of the controlled systems lead into a receiving container (30), immediately or after individual lines or all lines have been guided together, and a pump (4 to 6) and devices for keeping the primary pressure constant (38 to 43) are arranged in each instance between the storage containers and the associated controlled system upstream of the controlled system.

7. Device according to Claim 6,
**characterised in that**
the controlled system contains a mass-flow meter or a metering pump for the purpose of flow measurement.

8. Device according to one of Claims 6 or 7,
**characterised in that**
it comprises process control devices for matching the measurements and regulators with one another.

9. Device according to one of Claims 6 to 9,
**characterised in that**
it contains a mixing element (14), in particular a static mixer, in a line resulting from merging the lines (26) to (29).

## Revendications

1. Procédé en vue de la fabrication continue d'une solution aqueuse d'acide peroxy carboxylique en équilibre, comprenant la combinaison des composants individuels formant la solution aqueuse d'acide peroxy carboxylique en équilibre obtenue à partir de la série des acides carboxyliques inférieurs, en particulier de l'acide acétique, du peroxyde d'hydrogène aqueux, d'eau et de catalyseurs d'acides minéraux et la mise au repos du mélange de substances dans un récipient jusqu'à établissement d'une conversion souhaitée, **caractérisé en ce que** l'on prélève les composants individuels de réservoirs de réserve ou de réseaux de distribution et que l'on forme des courants continus des composants individuels, **en ce que** l'on conduit chaque courant de composant à travers un trajet de régulation comprenant un dispositif de mesure du débit en masse ou en volume et un organe de régulation en vue de la régulation du débit, **en ce que** l'on régule de manière proportionnelle aux quantités les courants quantitatifs des composants individuels en tenant compte du courant quantitatif d'un premier composant et **en ce que** l'on introduit les courants quantitatifs régulés des composants du mélange de substances immédiatement ou après une combinaison complète ou partielle des courants quantitatifs individuels dans un récipient de réception et **en ce que** l'on mesure le débit du courant global formé par les courants individuels ou la quantité globale des courants individuels ou partiels introduits dans le récipient et **en ce que** l'on compense avec la somme des courants individuels.

2. Procédé selon la revendication 1, **caractérisé en ce que** l'on utilise, en vue de la mesure du débit, un dispositif en vue de la mesure du débit en masse ou une pompe de dosage.

3. Procédé selon la revendication 1 ou 2, **caractérisé en ce que** l'on conduit, par l'intermédiaire des trajets de régulation, les courants prélevés des réservoirs de réserve à l'aide de pompes ou grâce à des pressions préalables présentes.

4. Procédé selon l'une quelconque des revendications 1 à 3, **caractérisé en ce que** l'on règle, à une valeur constante, en vue du maintien à un niveau constant des conditions de régulation, la pression préalable du courant de composant correspondant avant le trajet de régulation correspondant.

5. Procédé selon l'une quelconque des revendications 1 à 4, **caractérisé en ce que** l'on combine l'un après l'autre les courants de composant proportionnels quantitatifs et **en ce que** l'on procède à l'homogénéisation des courants individuels formés ou du courant global formé à l'aide d'organes de mélange, en particulier de malaxeurs statiques.

6. Dispositif en vue de la fabrication continue de solutions aqueuses d'acide peroxy carboxylique en équilibre selon l'une quelconque des revendications 1 à 5, comprenant des réservoirs de réserve ou des réseaux de distribution pour les composants individuels de la solution en équilibre, des dispositifs en vue du dosage des composants individuels et un récipient de réception, **caractérisé en ce qu'**un conduit partant de chaque réservoir de réserve (1, 2, 3) ou de chaque réseau de distribution (12) est formé en tant que trajet de régulation (22 à 25), **en ce que** chaque trajet de régulation présente un dispositif de mesure du débit (8 à 11) et un organe de régulation (34 à 37) en vue de la régulation du débit et **en ce qu'**une unité de régulation ayant des conduits de commande (31, 32, 33) rend possible un dosage proportionnel quantitatif des composants, et **en ce que** les conduits intercalés après les trajets de régulation (26 à 29) débouchent immédiatement ou après combinaison des conduits individuels ou de tous les conduits dans un récipient de réception (30) et **en ce qu'**entre les réservoirs de réserve et le trajet de régulation correspondant sont disposés, à chaque fois, une pompe (4 à 6) et des dispositifs en vue du maintien à un niveau constant de la pression préalable (38 à 43) avant le trajet de régulation.

7. Dispositif selon la revendication 6, **caractérisé en ce que** le trajet de régulation en vue de la mesure du débit contient un débitmètre massique ou une pompe de dosage.

8. Dispositif selon l'une quelconque des revendications 6 ou 7, **caractérisé en ce qu'**il présente, dans l'optique de l'accord réciproque des mesures et des régulateurs les unes avec les autres, des dispositifs en vue de la technique de la conduite de processus.

9. Dispositif selon l'une quelconque des revendications 6 à 8, **caractérisé en ce qu'**il présente, dans un conduit résultant de la combinaison des conduits (26) à (29), un organe de mélange (14), en particulier un malaxeur statique.
